# EUROPEAN PATENT APPLICATION

(11) **EP 4 445 931 A1**
(43) Date of publication of application: **16.10.2024**
(21) Application number: 23842602.7
(22) Date of filing: 10.02.2023
(51) Int. Cl.: A61M 5/34

(54) **NEEDLE HUB AND SYRINGE USING SAME**

(30) Priority: 20.07.2022 JP 2022115785
(71) Applicant: TSK Laboratory, Japan, Tochigi 328-0002 (JP)
(72) Inventor: KAWASHIMA, Ken, Tochigi-shi, Tochigi 328-0002 (JP); KOBAYASHI, Hirotsugu, Tochigi-shi, Tochigi 328-0002 (JP); SHIBATA, Ryo, Tochigi-shi, Tochigi 328-0002 (JP); FUJISAWA, Toru, Tochigi-shi, Tochigi 328-0002 (JP)
(74) Representative: Serjeants LLP
(86) International application number: PCT/JP2023/004566
(87) International publication number: WO 2024/018664

(57) **Abstract**

In order to provide a needle hub that can be coupled to various syringes with a small dead space, there is provided a needle hub, which is formed of a thermoplastic resin having a tensile modulus of elasticity of 1400 MPa or more and 2500 MPa or less, and has a recessed portion having a conical trapezoidal shape to be fitted to a male Luer-slip connector formed of polypropylene as defined in ISO 80369-7: 2016, and in which a needle is provided on a front side of the recessed portion, wherein the recessed portion has a depth L of 7.384 mm or more and 7.496 mm or less, an inner diameter D of an opening portion of 4.316 mm or more and 4.376 mm or less, and an inner diameter narrows from the opening portion at an inclination of 6% with respect to the depth.

## Description

### TECHNICAL FIELD

The present invention relates to a needle hub for puncturing a subject and an injector using the same.

### BACKGROUND ART

Conventionally, needle hubs and syringes have been manufactured according to ISO standards. This has the advantage that product compatibility is increased and any injector assembly can be injected in the same way. Currently, one of the ISO standards for the needle hub and syringe connector is defined in ISO 80369-7: 2016. ISO 80369-7: 2016 defines a Luer-slip connector and a Luer-lock connector.

However, the injector conforming to the conventional standards has a large dead space where a medical solution remains. Eliminating the dead space of the injector to reduce the loss of expensive medical solution has a great advantage.

In order to eliminate the dead space of the needle hub and the injection barrel as defined in ISO 80369-7: 2016, for example, Patent Document 1 discloses a injector having a longitudinal axis, including an injector hub assembly having a longitudinal axis, in which the injection needle assembly includes an injection needle hub body, in which the injection needle hub body has a syringe connector for receiving an attachment portion of an injection barrel of the injector at a first end portion of the injection needle hub body, and further has an injection needle receiving portion at a second end portion opposite to the injection needle hub body, in which the injection needle hub assembly further includes an injection needle extending from the injection needle receiving portion, in which the syringe connector has a receiving opening portion having a substantially conical shape for receiving the attachment portion of the injector of the injection barrel at least at a distal end thereof, an inlet region of the receiving opening portion has a cross-sectional dimension (D) of approximately 4.270 mm to 4.315 mm, an inner peripheral wall of the receiving opening portion extending between a periphery of the inlet region and a periphery of an upper surface tapers from the inlet region towards the upper surface by 6%, and a length (A) of an opening portion measured along the longitudinal axis is from 3 mm to 7 mm, in which one end portion of the injection needle is received in the injection needle receiving portion and does not protrude into the opening portion, in which the injector includes the injection barrel, in which the injection barrel extends in the longitudinal axis direction between a proximal end and the distal end of the injector, and includes an attachment portion for attaching the injection needle hub assembly, in which the syringe further includes a plunger slidably received in the injection barrel, in which the plunger includes a sealing element, in which the sealing element is arranged at a distal end of the plunger and configured to be assembled at least inside the attachment portion, and in which a material of the injection needle hub body of the injection needle hub assembly has a higher hardness than a material of the injection barrel of the injector, at least a material of the attachment portion of the injection barrel of the injector.

### PRIOR ART DOCUMENT

### PATENT DOCUMENT

Patent Document 1: Japanese Patent No. 6864475

### SUMMARY OF THE INVENTION

### PROBLEMS TO BE SOLVED BY THE INVENTION

However, in the injector, if the coupling force between the injection needle hub body and the syringe is not optimized, there is a possibility that the injection needle hub body and the syringe are disassembled during injection due to the injection pressure of the medical solution. In particular, in a case where a medical solution having a high viscosity is used, the possibility increases. Here, the high viscosity is assumed to be higher in viscosity than water. In order to strengthen the coupling so that the needle hub and the syringe are not disassembled, it is conceivable to use a Luer-lock connector, but the structure becomes complicated and the cost increases. In order to reduce the cost, it is better to employ a Luer-slip type.

Therefore, an object of the present invention is to provide a needle hub having a small dead space to be fitted to a male Luer-slip connector as defined in ISO 80369-7: 2016 and capable of increasing the coupling force, and to configure an injector having a small dead space with syringes including various male Luer-slip connectors as defined in ISO 80369-7: 2016. It is also an object to provide a needle hub to be fitted to a male Luer-slip connector as defined in ISO 80369-7: 2016, which also couples with a male Luer-lock connector as defined in ISO 80369-7: 2016, which can be broadly compatible.

It is also an object to provide an injector composed of the needle hub and a syringe having a male Luer-slip connector as defined in ISO 80369-7: 2016 to reduce the cost, reduce the dead space, and increase the coupling force. Furthermore, it is an object to provide an injector composed of the needle hub and a syringe having a male Luer-lock connector as defined in ISO 80369-7: 2016.

### SOLUTIONS TO THE PROBLEMS

A needle hub of the present invention has a recessed portion having a conical trapezoidal shape to be fitted to a male Luer-slip connector as defined in ISO 80369-7: 2016, in which a needle is provided on a front side of the recessed portion,
in which the recessed portion has a depth L of 7.384 mm or more and 7.496 mm or less, an inner diameter D of an opening portion of 4.316 mm or more and 4.376 mm or less, and an inner diameter narrows from the opening portion at an inclination of 6% with respect to the depth. At this time, the needle hub is preferably formed of a thermoplastic resin having a tensile modulus of elasticity of 1400 MPa or more and 2500 MPa or less, and the male Luer-slip connector is preferably formed of a thermoplastic resin such as polypropylene. Specifically, the needle hub is preferably formed of a thermoplastic resin such as polycarbonate, polypropylene, a cyclic olefin polymer (COP), or Tritan (registered trademark) which is one of copolyester resins.

According to the present invention, it is possible to reduce the dead space and obtain the needle hub having a large coupling force with the syringe. Therefore, it is possible to provide the needle hub that can be coupled to various syringes with a small dead space. In addition, the needle hub of the present invention can be used for injection of an expensive medical solution and a medical solution having high viscosity.

Further, in the needle hub of the present invention, a thread is provided on an outer surface of the needle hub, and the recessed portion and the thread of the needle hub are engageable with the male Luer-lock connector as defined in ISO 80369-7: 2016.

According to the present invention, a Luer-lock needle hub compatible with a Luer-slip type can be obtained.

Further, an injector of the present invention includes:
the needle hub described above;
a syringe having a male Luer-slip connector as defined in ISO 80369-7: 2016; and
a plunger including a gasket at a tip,
in which a shape of an outer surface of the gasket coincides with a shape of an inner surface of a tip of the syringe, and
a residual amount of the medical solution in the injector is 2 µL or more and 50 µL or less.

According to the present invention, it is possible to reduce the cost, reduce the dead space, and obtain the injector having a large coupling force. Therefore, the injector of the present invention can be used for injection of an expensive medical solution and a medical solution having high viscosity.

Further, an injector of the present invention includes:
the needle hub described above;
a syringe having a male Luer-lock connector as defined in ISO 80369-7: 2016; and
a plunger including a gasket at a tip,
in which a shape of an outer surface of the gasket coincides with a shape of an inner surface of a tip of the syringe, and
a residual amount of the medical solution in the injector is 2 µL or more and 50 µL or less.

According to the present invention, a Luer-lock injector compatible with a Luer-slip type can be obtained.

### EFFECTS OF THE INVENTION

The present invention provides the needle hub having a small dead space to be fitted to the male Luer-slip connector as defined in ISO 80369-7: 2016 and capable of increasing the coupling force, and the injector having a small dead space with the syringes including various male Luer-slip connectors as defined in ISO 80369-7: 2016 can be configured. The present invention also provides the needle hub to be fitted to the male Luer-slip connector as defined in ISO 80369-7: 2016, which also couples with the male Luer-lock connector as defined in ISO 80369-7: 2016, which can be broadly compatible.

The present invention also provides the injector composed of the needle hub and the syringe having the male Luer-slip connector as defined in ISO 80369-7: 2016, which can reduce the cost, reduce the dead space, and increase the coupling force. Furthermore, the present invention provides the injector composed of the needle hub and the syringe having the male Luer-lock connector as defined in ISO 80369-7: 2016.

### BRIEF DESCRIPTION OF THE DRAWINGS

Fig. 1 is a view showing an overview of an injector according to an embodiment of the present invention.
Fig. 2 is a cross-sectional view of a needle hub according to the embodiment of the present invention.
Fig. 3 is a view illustrating an outline of the needle hub according to the embodiment of the present invention.
Fig. 4 is a cross-sectional view of the injector using the needle hub and a Luer-slip connector according to the embodiment of the present invention.
Fig. 5 is a cross-sectional view of the injector using the needle hub and the Luer-lock connector according to the embodiment of the present invention.
Fig. 6 illustrates a distance A between a tip surface of the Luer-slip connector and a bottom surface of a recessed portion of the needle hub when the Luer-slip connector is pushed into the recessed portion of the needle hub to be in the fixed state.

### DETAILED DESCRIPTION

Hereinafter, various embodiments for carrying out the present invention will be described with reference to the drawings. A needle hub and an injector described below are intended to embody the technical idea of the present disclosure, and the present disclosure is not limited to the following unless otherwise specified. In consideration of the description of the main points or ease of understanding, the same reference numerals are used in different drawings for convenience. In particular, the same operation and effect by the same configuration will not be sequentially mentioned for each embodiment or each example. The sizes, positional relationships, and the like of the members shown in the drawings may be exaggerated for clarity of description.

### (Description of Injector according to Embodiment of Present Invention)

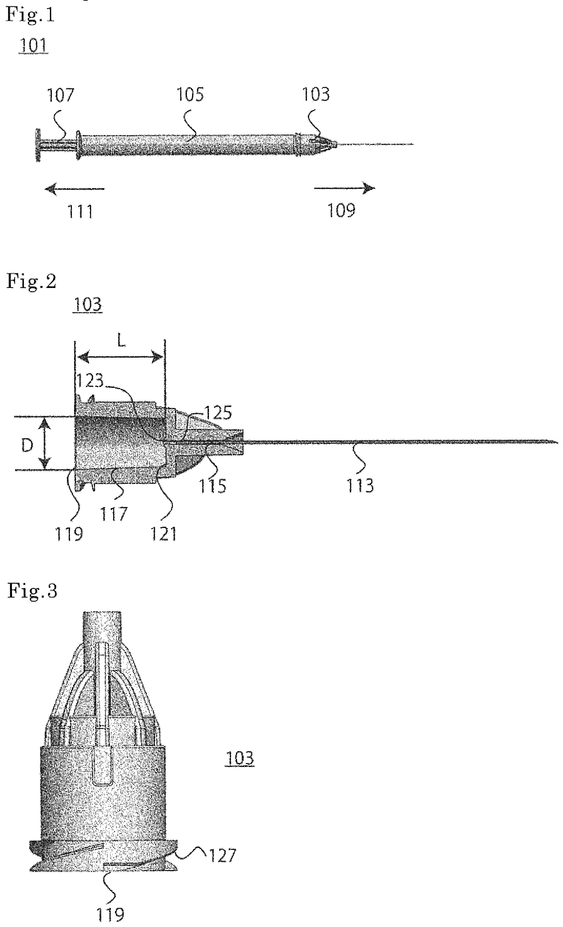

Fig. 1 is a view showing an overview of an injector according to an embodiment of the present invention. The injector according to the embodiment of the present invention will be described with reference to Fig. 1.

In Fig. 1, an arrow direction 109 in which a needle of the injector is located is referred to as a front end, a front end side, a front end direction, a front end portion, a front side, and the like, and an arrow direction 111 in which a plunger is inserted into the syringe is referred to as a rear end, a rear end side, a rear end direction, a rear end portion, a rear side, and the like.

As shown in Fig. 1, an injector 101 using a Luer-slip connector includes a needle hub 103 with a needle 113 (shown later) at a front end, a syringe 105 in which a medical solution to be injected is stored, and a plunger 107 that is attached to the syringe 105 and pushes the medical solution of the syringe 105. In the injector 101 shown in Fig. 1, the needle hub 103 is fitted and attached to the front end of the syringe 105, and is coupled by a Luer-slip connector coupled by a frictional force. In the case of a Luer-slip connector, this frictional force should be stronger than the injection pressure of the medical solution.

On the other hand, in the Luer-lock connector, as shown later, a male thread on an outer surface of the needle hub 103 is coupled to a female thread of the syringe. The Luer-lock connector is suitable for injecting a medical solution having a high viscosity because not only a frictional force between the tip of the syringe and the needle hub but also a coupling force is strong because the connector is coupled by fitting of the male thread and the female thread. However, since the structure becomes complicated, the cost may increase.

The syringe 105 has a hollow cylindrical shape and stores a medical solution. The syringe 105 has a tip (shown below) 135, 147 at the front end to be fitted to the needle hub, the center of which has a through hole. The medical solution having passed through the through hole of the fitting portion passes through the needle.

The plunger 107 is also called a pusher. A gasket 133,145 shown later is attached to the front end of the plunger 107. With the gasket 133,145 attached to the front end, the plunger 107 can function as a piston that pushes the medical solution in the syringe 105 to the front end without leaking.

The needle 113 is preferably formed of metal. The needle hub 103 is preferably formed of a thermoplastic resin such as polycarbonate, polypropylene, a cyclic olefin polymer (COP), or Tritan (registered trademark) which is one of copolyester resins. The syringe 105 is preferably formed of a resin such as polypropylene. The plunger 107 is preferably formed of a resin such as polypropylene or ABS. In addition, it is preferable that the entire gasket 133,145 is formed of a resin such as silicon or elastomer.

### (Description of Needle Hub according to Embodiment of Present Invention)

Fig. 2 is a cross-sectional view of the needle hub according to the embodiment of the present invention. Fig. 3 is a view showing an outline of the needle hub according to the embodiment of the present invention. With reference to Figs. 2 and 3, the needle hub according to the embodiment of the present invention will be described.

As shown in Fig. 2, the needle hub 103 according to the embodiment of the present invention has a recessed portion 117 having a conical trapezoidal shape to be fitted to a male Luer-slip connector as defined in ISO 80369-7: 2016, and the needle 113 provided on the front side of the recessed portion is attached.

The standard of ISO 80369-7: 2016 is a standard for adapting needle hubs and syringes, each made by another company, and is used particularly for injectors. Although compatibility is maintained by this standard, it is necessary to optimize each of the needle hub and the syringe to have a desired coupling force.

The needle 113 is inserted into a needle receiving portion 115 and fixed with an adhesive. The needle 113 is not completely inserted into the needle receiving portion 115, but a space 125 is formed. The space 125 is a cylindrical space having a diameter corresponding to the outer diameter of the needle 113, which varies depending on the degree of insertion of the needle 113. This also becomes a dead space of the medical solution, and thus it is preferable to make the space as small as possible.

The recessed portion 117 has an opening portion 119 for receiving the connector and a bottom surface 121 for terminating the connector. A distance between the opening portion 119 and the bottom surface 121, that is, a depth L of the recessed portion 117 is 7.384 mm or more and 7.496 mm or less. The opening portion 119 and the bottom surface 121 form concentric circles on the central axis in the longitudinal direction of the injector. An inner diameter D of the opening portion 119 is 4.316 mm or more and 4.376 mm or less. The opening of the recessed portion 117 narrows with an inclination of 6% from the opening portion 119 toward the bottom surface 121. In this way, the needle hub 103 is strongly coupled with the male Luer-slip connector of ISO 80369-7: 2016.

The above numerical ranges of the depth L and the inner diameter D of the recessed portion 117 will be described in more detail. When the male Luer-slip connector is formed of polypropylene and the needle hub 103 is formed of a thermoplastic resin such as polycarbonate, polypropylene, a cyclic olefin polymer (COP), or Tritan (registered trademark), both the male Luer-slip connector and the needle hub 103 are deformed during fitting, and the degree of deformation differs between the connector and the needle hub. At this time, it is considered that the tensile modulus of elasticity or the flexural modulus of elasticity of the resin material constituting the member is effective as an index of the degree of deformation.

When the Luer-slip connector is pushed into and attached to the recessed portion of the needle hub 103, since the outer surface of the Luer-slip connector and the inner surface of the recessed portion of the needle hub have the tapered surface, a high surface pressure can be obtained by the wedge effect, and the coupling force between the Luer-slip connector and the needle hub 103 is increased. Furthermore, when the hardness differs between the Luer-slip connector and the needle hub 103, one of the Luer-slip connector and the needle hub 103 is deformed more than the other, and thus, the outer surface of the Luer-slip connector and the inner surface of the recessed portion of the needle hub are in close contact with each other strongly, and a higher surface pressure and a stronger coupling force can be obtained.

On the other hand, both the Luer-slip connector and the needle hub 103 have dimensional tolerance, and have a certain degree of dimensional variation for each product. Furthermore, since the frictional state at the time of fitting is not constant, when the Luer-slip connector is pushed into the recessed portion 117 of the needle hub 103, the movement of the Luer-slip connector is stopped, and the final pushing amount in the fixed state slightly varies. It is difficult to obtain such variation in the pushing amount by arithmetic calculation.

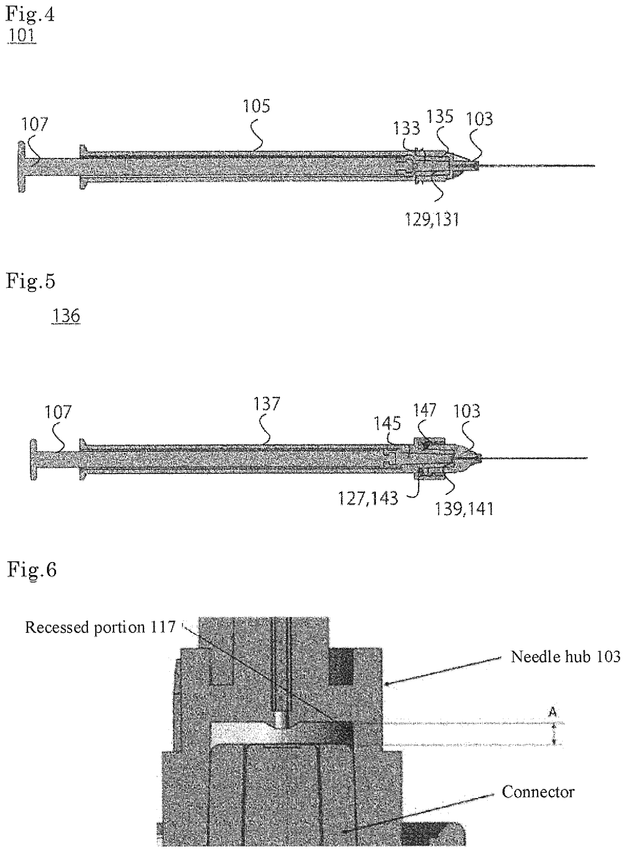

Fig. 6 shows a distance A between the tip surface of the Luer-slip connector and the bottom surface of the recessed portion 117 of the needle hub 103 when the Luer-slip connector is pushed into the recessed portion 117 of the needle hub 103 to be in the fixed state.

From the viewpoint of reducing the dead space, it is preferable to reduce the distance A between the tip surface of the Luer-slip connector and the bottom surface of the recessed portion 117 of the needle hub 103. On the other hand, when the distance A decreases, the distance A slightly varies depending on the product, so that there is a possibility that the tip surface of the Luer-slip connector and the bottom surface of the recessed portion 117 of the needle hub 103 abut on each other (that is, the distance A = 0).

When the tip surface of the Luer-slip connector and the bottom surface of the recessed portion 117 of the needle hub 103 abut on each other, both cannot move relative to each other, so that a surface pressure due to a wedge effect between the outer surface of the Luer-slip connector and the inner surface of the recessed portion 117 of the needle hub 103 cannot be obtained. For this reason, the sealability between the outer surface of the Luer-slip connector and the inner surface of the recessed portion 117 of the needle hub 103 is deteriorated, and a problem such as leakage of the injection solution occurs.

As described above, it is necessary to solve the problem that it is difficult to suppress the dead space by reducing the distance A as much as possible in a state where the predetermined distance A is reliably secured and the strong coupling force between the male Luer-slip connector and the needle hub 103 is secured even if there are some variations in the product dimensions and the friction state at the time of fitting. In order to solve such a problem that cannot be handled by simple desk calculation, the inventors prototyped needle hubs 103 formed of various materials having various elastic moduli and having recesses of various dimensions, while considering the elastic modulus of the material as an index of the degree of deformation, and performed a test of fitting with the Luer-slip connector defined in ISO 80369-7: 2016, to find the above numerical ranges of the depth L and the inner diameter D of the recessed portion 117. Hereinafter, test results will be described as first to fourth examples.

The inner surface of the recessed portion 117 has a smooth curved surface without unevenness. That is, the curved surface generates friction between the male Luer-slip connector and the recessed portion 117 as a whole, instead of reducing the opening by a protrusion or the like to increase the frictional force. In this manner, the needle hub 103 is further strongly coupled to the male Luer-slip connector.

A protruding portion 123 is formed at a substantially central position of the bottom surface 121 of the recessed portion 117 in plan view. The protruding portion 123 is engaged with the center of the male Luer-slip connector to position the central axis. In addition, when the shape of the needle hub 103 is changed, it is conceivable that the dead space changes in the space between the needle hub 103 and the Luer-type connector. Therefore, the protruding portion 123 reduces the dead space between the needle hub 103 and the male Luer-slip connector.

As shown in Fig. 3, the needle hub 103 includes an male thread 127 on an outer surface near the opening portion 119. The thread of this male thread can be engaged with the male Luer-lock connector as defined in ISO 80369-7: 2016 and a female thread 143 (shown later). By the engagement between the male thread 127 and the female thread 143 and the engagement between the recessed portion 117 of the needle hub 103 and the tip of the male Luer-lock connector, the needle hub and the male Luer-lock connector can be firmly coupled. As described above, the needle hub of the present embodiment can be engaged not only with the male Luer-slip connector as defined in ISO 80369-7: 2016 but also with the male Luer-lock connector as defined in ISO 80369-7: 2016, and the compatibility can be expanded.

### (Description of Injector including Syringe having Needle Hub and Male Luer-slip Connector of Present Embodiment)

Fig. 4 is a cross-sectional view of the injector using the needle hub and the Luer-slip connector according to the embodiment of the present invention. With reference to Fig. 4, the injector using the needle hub and the Luer-slip connector according to the embodiment of the present invention will be described.

As shown in Fig. 4, the injector 101 using the needle hub 103 and the Luer-slip connector of the embodiment of the present invention includes the plunger 107, the gasket 133 attached to the plunger tip, the syringe 105, and the needle hub 103 fitted and attached to the tip 135 of the syringe 105.

The male Luer-slip connector and the needle hub 103 as defined in ISO 80369-7: 2016 are coupled only by a frictional force of a smooth curved surface.

The shape of the outer surface 129 of the gasket 133 preferably coincides with the shape of the inner surface 131 of the tip 135 of the syringe 105. In this way, for the dead space, it is only necessary to consider the space between the syringe 105 and the needle hub 103, the inside of the needle, and the space 125 between the needle hub 103 and the needle.

In this way, it is possible to provide the injector 101 having a low dead space. The low dead space means that the residual amount of the medical solution, that is, the volume of these spaces is 50 µL or less, preferably 20 µL or less, and more preferably 14 µL or less.

### (Description of Injector including Syringe having Needle Hub and Male Luer-lock Connector of Present Embodiment)

Fig. 5 is a cross-sectional view of the injector using the needle hub and the Luer-lock connector according to the embodiment of the present invention. With reference to Fig. 5, the injector using the needle hub and the Luer-lock connector according to the embodiment of the present invention will be described.

As shown in Fig. 5, an injector 136 using the needle hub 103 and the Luer-lock connector according to the embodiment of the present invention includes the plunger 107, the gasket 145 attached to the plunger tip, a syringe 137, and the needle hub 103 fitted and attached to the tip 147 of the syringe 137.

The male Luer-lock connector and the needle hub 103 as defined in ISO 80369-7: 2016 are tightly coupled by a frictional force of a smooth curved surface and screwing of the female thread 143 and the male thread 127.

The shape of the outer surface 139 of the gasket 145 preferably coincides with the shape of the inner surface 141 of the tip 147 of the syringe 137. In this way, for the dead space, it is only necessary to consider the space between the syringe 137 and the needle hub 103, the inside of the needle, and the space 125 between the needle hub 103 and the needle.

In this way, it is possible to provide the injector 136 having a low dead space. Similarly to the male Luer-slip connector as defined in ISO 80369-7: 2016, the low dead space means that the residual amount of the medical solution, that is, the volume of these spaces is 50 µL or less, preferably 20 µL or less, and more preferably 14 µL or less.

### EXAMPLES

As examples, in consideration of the tensile modulus of elasticity as an index of the degree of deformation of the needle hub, needle hubs were prototyped using polycarbonate (first example) having a tensile modulus of elasticity of 2500 MPa, polypropylene (second example 2) having a tensile modulus of elasticity of 1400 MPa, a cyclic olefin polymer (COP) (third example 3) having a tensile modulus of elasticity of 2200 MPa, and Tritan (registered trademark) (fourth example) having a tensile modulus of elasticity of 1550 MPa as materials, and the following tests were performed.

### (First Example 1)

By changing the depth L and the inner diameter D of the opening portion, a needle hub 103 made of polycarbonate (tensile modulus of elasticity: 2500 MPa, flexural modulus of elasticity: 2100 MPa) having a recessed portion with an inclination of 6% in the opening spread with respect to the depth was prototyped, and fitted to syringes made of polypropylene manufactured by four major companies, and the dead spaces and the separation resistances were compared. The results are shown in Table 1.

**[Table 1]**

| Table of dimensions of recessed portion of needle hub, dead space, and separation resistance [mm] | | | | | | | | | | |
|---|---|---|---|---|---|---|---|---|---|---|
| | Depth L (mm) | | Opening portion diameter D (mm) | | Residual amount of medical solution (µL) | | Separation resistance (N) | | | |
| | Minimum | Maximum | Minimum | Maximum | Minimum | Maximum | Syringe of A company | Syringe of B company | Syringe of C company | Syringe of D company |
| Example 1 | 7.384 | 7.409 | 4.371 | 4.376 | 7 | 12 | ○ | ○ | ○ | ○ |
| Example 2 | 7.429 | 7.457 | 4.316 | 4.371 | 7 | 14 | ○ | ○ | ○ | ○ |
| Example 3 | 7.473 | 7.496 | 4.317 | 4.358 | 8 | 14 | ○ | ○ | ○ | ○ |
| Comparative Example 1 | 7.380 | 7.392 | 4.552 | 4.555 | 2 | 9 | 12.1 | 12.1 | 19.5 | 6.1 |
| Comparative Example 3 | 7.393 | 7.416 | 4.554 | 4.559 | 5 | 13 | ○ | 18.8 | ○ | 10.4 |
| Comparative Example 3 | 7.428 | 7.442 | 4.585 | 4.589 | 6 | 13 | 19.9 | 18.8 | ○ | 13.2 |
| Comparative Example 4 | 7.479 | 7.492 | 4.585 | 4.593 | 4 | 13 | 20.6 | 19.5 | 22.9 | 14.9 |
| Conventional product | 6.1 | 6.2 | 4.27 | 4.315 | 4 | 10 | 4.5 | 5.1 | 11.9 | 1.6 |

As the separation resistance, a separation force of 23 N is held in the axial direction for 10 seconds, a needle hub that is not separated from the syringe is ∘, and when a needle hub is separated, the force in the axial direction at the time of separation is described. In a conventional product, the length L was 6.1 mm or more and 6.2 mm or less, and the inner diameter D of the opening portion was 4.27 mm or more and 4.315 mm or less, and the conventional product was separated without achieving a certain separation force with the syringe of each company. It is considered that in the conventional product, the depth L was short, a sufficient frictional force was not obtained, and the coupling force was weak, which led to separation.

On the other hand, in Comparative Examples 1 to 4 in which the length of the depth L was increased to 7.380 mm or more and 7.492 mm or less, the coupling force was stronger than that of the conventional product, but some comparative examples could not achieve a certain separation force with the syringe of each company. Comparative Examples 1 to 4 have an opening portion inner diameter D of 4.552 mm or more and 4.593 mm or less, and correspond to that obtained by extending the recessed portion to the rear end side from the conventional product. Therefore, it is considered that the coupling force of Comparative Examples 1 to 4 was weak because the opening portion diameter was increased.

On the other hand, in Examples 1 to 3, the depth L was 7.384 mm or more and 7.496 mm or more, which was larger than that of the conventional product, and the opening portion inner diameter D was 4.316 mm or more and 4.376 mm or less, which was smaller than those of comparative examples. Examples 1 to 3 correspond to that obtained by extending the recessed portion to the front end side from the conventional product. In Examples 1 to 3, a sufficient coupling force was obtained, and Examples 1 to 3 were not separated with a constant separation force with the syringe of each company.

In Examples 1 to 3, the dead space was 14 µL or less, and the dead space could be reduced to a level comparable to that of Comparative Examples and the conventional product.

The present invention provides the needle hub having a small dead space to be fitted to the male Luer-slip connector as defined in ISO 80369-7: 2016 and capable of increasing the coupling force, and the injector having a small dead space with the syringes including the various male Luer-slip connectors as defined in ISO 80369-7: 2016 can be configured. The present invention also provides the needle hub to be fitted to the male Luer-slip connector as defined in ISO 80369-7: 2016, which also couples with the male Luer-lock connector as defined in ISO 80369-7: 2016, which can be broadly compatible.

The present invention also provides the injector composed of the needle hub and the syringe having the male Luer-slip connector as defined in ISO 80369-7: 2016, which can reduce the cost, reduce the dead space, and increase the coupling force. Furthermore, the present invention provides the injector composed of the needle hub and the syringe having the male Luer-lock connector as defined in ISO 80369-7: 2016.

The needle hub and the injector of the present invention can be used for injection of an expensive medical solution and a medical solution having high viscosity.

### (First Example 2)

Next, as a first example 2, a needle hub made of polycarbonate (tensile modulus of elasticity: 2500 MPa, flexural modulus of elasticity: 2100 MPa) was prototyped, fitted to the syringes made of polypropylene manufactured by four major companies, and subjected to a test for measuring the distance A shown in Fig. 6.

As the needle hub, a conventional hub which is a conventional needle hub defined in ISO 80369-7: 2016, a first LD hub in which the entire length of the depth of the recessed portion is shortened to 6.1 mm in the drawing dimension so as to reduce the dead space as much as possible, and a second LD hub in which the entire length of the depth of the recessed portion is extended to the side where the inner diameter is narrowed by approximately 1.3 mm in the drawing dimension as compared with the first LD hub were prepared. The second LD hub corresponds to the present invention, and is formed such that the depth L of the recessed portion is 7.384 mm or more and 7.496 mm or less, the inner diameter D of the opening portion is 4.316 mm or more and 4.376 mm or less, and the inner diameter narrows from the opening portion at an inclination of 6% with respect to the depth.

The conventional hub, the first LD hub, and the second LD hub were fitted to the Luer-slip connector of each company, and the distance A (see Fig. 6) between the tip surface of the Luer-slip connector and the bottom surface of the recessed portion of the needle hub was measured. Two samples (sample 1 and sample 2) were prepared for each of the conventional hub, the first LD hub, and the second LD hub, and fitted to the Luer-slip connectors of the four main manufacturers to measure the distance A.

More specifically, one side of the hub upper surface was cut to expose the syringe tip, measurement was performed with a measurement microscope, and the shortest distance between the tip surface of the Luer-slip connector and the bottom surface of the recessed portion of the needle hub was defined as a distance A. The measurement results are shown in Table 2.

**[Table 2]**

| Table of distance A after connector and needle hub are brought into fixed state [mm] | | | | | | |
|---|---|---|---|---|---|---|
| Connector | Syringe of A company | | | Syringe of B company | | |
| Needle hub | Conventional product | First LD | Second LD | Conventional product | First LD | Second LD |
| Measurement result of first sample | 4.448 | 0.000 | 0.687 | 4.705 | 0.000 | 0.779 |
| Measurement result of second sample | 4.479 | 0.000 | 0.666 | 4.562 | 0.069 | 0.808 |
| Average value | 4.463 | 0.000 | 0.677 | 4.633 | 0.034 | 0.793 |
| Taper length (drawing value) | 10.700 | 6.100 | 7.400 | 10.700 | 6.100 | 7.400 |

| Connector | Syringe of C company | | | Syringe of D company | | |
|---|---|---|---|---|---|---|
| Needle hub | Conventional product | First LD | Second LD | Conventional product | First LD | Second LD |
| measurement result of first sample | 4.894 | 0.059 | 0.836 | 4.790 | 0.000 | 0.561 |
| Measurement Result of Second sample | 4.721 | 0.000 | 0.987 | 4.221 | 0.000 | 0.837 |
| Average value | 4.808 | 0.030 | 0.912 | 4.505 | 0.000 | 0.699 |
| Taper length (drawing value) | 10.700 | 6.100 | 7.400 | 10.700 | 6.100 | 7.400 |

As is clear from Table 2, it was clarified that in the conventional hub, the distance A exceeds 4 mm, and a considerably large dead space is generated. In the first LD hub, the distance A was less than 0.1 mm, and it was clarified that the dead space was very small. However, in some measurement results, the distance A = 0. That is, it has been found that there is a possibility that the tip surface of the Luer-slip connector and the bottom surface of the recessed portion of the first LD hub abut on each other and a sufficient coupling force cannot be obtained by the Luer-slip connector and the needle hub.

Although the distance A of the second LD hub is larger than that of the first LD hub, the distance A is less than 1 mm in each of the first LD hub and the second LD hub, and thus it was clarified that the dead space is sufficiently small. Further, it has been found that there is no possibility that the tip surface of the Luer-slip connector and the bottom surface of the recessed portion of the second LD hub abut on each other even if the distance A is larger than 0.5 mm and some variation occurs, and a sufficient coupling force can be obtained by the Luer-slip connector and the needle hub.

As described above, in the recessed portion of the needle hub made of polycarbonate having a tensile modulus of elasticity of 2500 MPa, the depth L of the recessed portion is 7.384 mm or more and 7.496 mm or less, the inner diameter D of the opening portion is 4.316 mm or more and 4.376 mm or less, and the inner diameter is formed to be narrowed from the opening portion at an inclination of 6% with respect to the depth, whereby it has been demonstrated for the first time that the distance A is reliably secured, a strong coupling force between the male Luer-slip connector and the needle hub is secured, and a dead space can be sufficiently suppressed.

### (Second Example)

Next, even when the needle hub was formed of polypropylene, it was confirmed whether or not the strong coupling force between the male Luer-slip connector and the needle hub was secured and the dead space could be sufficiently suppressed as in the first example.

As a second Example, a result of a fitting test of the Luer-slip connector and the needle hub in a case where the needle hub is formed of polypropylene will be described. A needle hub made of polypropylene was prototyped, and a fitting test was performed by combining the needle hub with the syringes made of polypropylene manufactured by four major companies similar to that of the first example.

Examples 1 to 3, which are the three prototyped needle hubs, correspond to that obtained by extending the recessed portion to the front end side from the conventional product, and are formed such that the depth L of the recessed portion is 7.384 mm or more and 7.496 mm or less, the inner diameter D of the opening portion is 4.316 mm or more and 4.376 mm or less, and the inner diameter narrows from the opening portion at an inclination of 6% with respect to the depth.

The test method is similar to that of the first example except for the material of the prototyped needle hub, and thus, further description thereof is omitted.

Examples 1 to 3, which are the prototyped needle hub made of polypropylene (tensile modulus of elasticity: 1400 MPa, flexural modulus of elasticity: 1410 MPa), were fitted to the syringes made of polypropylene manufactured by four major companies, and the dead spaces and the separation resistances were compared. The results are shown in Table 3. In addition, Examples 1 to 3 and the syringes made of polypropylene manufactured by four major companies were fitted to each other, and the distance A was measured. The results are shown in Table 4.

**[Table 3]**

| Table of dimensions of recessed portion of needle hub, dead space, and separation resistance [mm] | | | | | | | | | | |
|---|---|---|---|---|---|---|---|---|---|---|
| | Depth L (mm) | | Opening portion diameter D (mm) | | Residual amount of medical solution (µL) | | Separation resistance | | | |
| | Minimum | Maximum | Minimum | Maximum | Minimum | Maximum | Syringe of A company | Syringe of B company | Syringe of C company | Syringe of D company |
| Example 1 | 7.358 | 7.358 | 4.266 | 4.266 | 7 | 11 | ○ | ○ | ○ | ○ |
| Example 2 | 7.405 | 7.405 | 4.274 | 4.274 | 7 | 11 | ○ | ○ | ○ | ○ |
| Example 3 | 7.456 | 7.456 | 4.269 | 4.269 | 8 | 15 | ○ | ○ | ○ | ○ |

As is clear from Table 3, even in Examples 1 to 3 made of polypropylene, a sufficient coupling force was obtained, and Examples 1 to 3 were not separated with a constant separation force with the syringe of each company. In Examples 1 to 3, the dead space was 15 µL or less, and it was demonstrated that the dead space can be sufficiently reduced.

**[Table 4]**

| Table of distance A after connector and needle hub are brought into fixed state [mm] | | | | |
|---|---|---|---|---|
| | Syringe of A company | Syringe of B company | Syringe of C company | Syringe of D company |
| Example 1 | 1.202 | 1.366 | 1.259 | 1.410 |
| Example 2 | 1.238 | 1.575 | 1.101 | 1.357 |
| Example 3 | 1.120 | 1.454 | 1.237 | 0.994 |

As is clear from Table 4, in Examples 1 to 3, the distance A was less than 1.6 mm, and it was clarified that the dead space was sufficiently small. Further, it has been found that there is no possibility that the tip surface of the Luer-slip connector and the bottom surface of the recessed portion of the needle hub abut on each other even if the distance A is larger than 0.9 mm and some variation occurs, and a sufficient coupling force can be obtained by the Luer-slip connector and the needle hub.

As described above, in the recessed portion of the needle hub made of propolypropylene having a tensile modulus of elasticity of 1400 MPa, the depth L of the recessed portion is 7.384 mm or more and 7.496 mm or less, the inner diameter D of the opening portion is 4.316 mm or more and 4.376 mm or less, and the inner diameter is formed to be narrowed from the opening portion at an inclination of 6% with respect to the depth, whereby it has been demonstrated that the distance A is reliably secured, a strong coupling force between the male Luer-slip connector and the needle hub is secured, and a dead space can be sufficiently suppressed.

### (Third example)

Next, even when the needle hub was formed of a cyclic olefin polymer (COP), it was confirmed whether or not the strong coupling force between the male Luer-slip connector and the needle hub was secured and the dead space could be sufficiently suppressed as in the first and second examples.

As a third example, a result of a fitting test of a Luer-slip connector and a needle hub in a case where the needle hub is formed of a cyclic olefin polymer (COP, tensile modulus of elasticity: 2200 MPa, flexural modulus of elasticity 2100: MPa) will be described. A needle hub made of a cyclic olefin polymer (COP) was prototyped, and a fitting test was performed by combining the needle hub with the syringes made of polypropylene manufactured by four major companies similar to that of the first and second examples.

Examples 1 and 2, which are the three prototyped needle hubs, correspond to that obtained by extending the recessed portion to the front end side from the conventional product, and are formed such that the depth L of the recessed portion is 7.384 mm or more and 7.496 mm or less, the inner diameter D of the opening portion is 4.316 mm or more and 4.376 mm or less, and the inner diameter narrows from the opening portion at an inclination of 6% with respect to the depth.

The test method is similar to that of the first and second examples except for the material of the prototyped needle hub, and thus, further description thereof is omitted.

Examples 1 and 2, which are the prototyped needle hubs made of a cyclic olefin polymer (COP), were fitted to the syringes made of polypropylene manufactured by four major companies, and the dead spaces and the separation resistances were compared. The results are shown in Table 5. In addition, Examples 1 and 2 and the syringes made of polypropylene manufactured by four major companies were fitted to each other, and the distance A was measured. The results are shown in Table 6.

**[Table 5]**

| Table of dimensions of recessed portion of needle hub, dead space, and separation resistance [mm] | | | | | | | | | | |
|---|---|---|---|---|---|---|---|---|---|---|
| | Taper length (mm) | | Opening portion diameter (mm) | | Residual amount of medical solution (µL) | | Separation resistance | | | |
| | min | max | min | max | min | max | Syringe of A company | Syringe of B company | Syringe of C company | Syringe of D company |
| Example 1 | 7.411 | 7.411 | 4.306 | 4.306 | 1 | 10 | ○ | ○ | ○ | ○ |
| Example 2 | 7.463 | 7.463 | 4.307 | 4.307 | 1 | 10 | ○ | ○ | ○ | ○ |

As is clear from Table 5, even in Examples 1 and 2 made of a cyclic olefin polymer (COP), a sufficient coupling force was obtained, and the needle hubs were not separated with a constant separation force with the syringe of each company. In Examples 1 and 2, the dead space was 10 µL or less, and it was demonstrated that the dead space can be sufficiently reduced.

**[Table 6]**

| Table of distance A after connector and needle hub are brought into fixed state [mm] | | | | |
|---|---|---|---|---|
| | Syringe of A company | Syringe of B company | Syringe of C company | Syringe of D company |
| Example 1 | 0.902 | 0.939 | 1.088 | 0.656 |
| Example 2 | 0.865 | 1.005 | 1.065 | 0.758 |

As is clear from Table 6, in Examples 1 and 2, the distance A was less than 1.1 mm, and it was clarified that the dead space was sufficiently small. Further, it has been found that there is no possibility that the tip surface of the Luer-slip connector and the bottom surface of the recessed portion of the needle hub abut on each other even if the distance A is larger than 0.6 mm and some variation occurs, and a sufficient coupling force can be obtained by the Luer-slip connector and the needle hub.

As described above, in the recessed portion of the needle hub made of a cyclic olefin polymer (COP) having a tensile modulus of elasticity of 2200 MPa, the depth L of the recessed portion is 7.384 mm or more and 7.496 mm or less, the inner diameter D of the opening portion is 4.316 mm or more and 4.376 mm or less, and the inner diameter is formed to be narrowed from the opening portion at an inclination of 6% with respect to the depth, whereby it has been demonstrated that the distance A is reliably secured, a strong coupling force between the male Luer-slip connector and the needle hub is secured, and a dead space can be sufficiently suppressed.

### (Fourth Example)

Next, even when the needle hub was formed of Tritan (registered trademark), it was confirmed whether or not the strong coupling force between the male Luer-slip connector and the needle hub was secured and the dead space could be sufficiently suppressed as in the first, second, and third examples.

As a fourth example, a result of a fitting test of a Luer-slip connector and a needle hub in a case where the needle hub is formed of Tritan (registered trademark) (tensile modulus of elasticity: 1550 MPa, flexural modulus of elasticity: 1550 MPa) will be described. A needle hub made of Tritan (registered trademark) was prototyped, and a fitting test was performed by combining the needle hub with the syringes made of polypropylene manufactured by four major companies similar to that of the first, second, and third examples.

Examples 1 and 2, which are the three prototyped needle hubs, correspond to that obtained by extending the recessed portion to the front end side from the conventional product, and are formed such that the depth L of the recessed portion is 7.384 mm or more and 7.496 mm or less, the inner diameter D of the opening portion is 4.316 mm or more and 4.376 mm or less, and the inner diameter narrows from the opening portion at an inclination of 6% with respect to the depth.

The test method is similar to that of the first, second, and third examples except for the material of the prototyped needle hub, and thus, further description thereof is omitted.

Examples 1 and 2, which are the prototyped needle hubs made of Tritan (registered trademark), were fitted to the syringes made of polypropylene manufactured by four major companies, and the dead spaces and the separation resistances were compared. The results are shown in Table 7. In addition, Examples 1 and 2 and the syringes made of polypropylene manufactured by four major companies were fitted to each other, and the distance A was measured. The results are shown in Table 8.

**[Table 7]**

| Table of dimensions of recessed portion of needle hub, dead space, and separation resistance [mm] | | | | | | | | | | |
|---|---|---|---|---|---|---|---|---|---|---|
| | Taper length (mm) | | Opening portion diameter (mm) | | Residual amount of medical solution (µL) | | Separation resistance | | | |
| | min | max | min | max | min | max | Syringe of A company | Syringe of B company | Syringe of C company | Syringe of D company |
| Example 1 | 7.406 | 7.406 | 4.313 | 4.313 | 1 | 8 | ○ | ○ | ○ | ○ |
| Example 2 | 7.463 | 7.463 | 4.312 | 4.312 | 5 | 14 | ○ | ○ | ○ | ○ |

As is clear from Table 7, even in Examples 1 and 2 made of Tritan (registered trademark), a sufficient coupling force was obtained, and the needle hubs were not separated with a constant separation force with the syringe of each company. In Examples 1 and 2, the dead space was 14 µL or less, and it was demonstrated that the dead space can be sufficiently reduced.

**[Table 8]**

| Table of distance A after connector and needle hub are brought into fixed state [mm] | | | | |
|---|---|---|---|---|
| | Syringe of A company | Syringe of B company | Syringe of C company | Syringe of D company |
| Example 1 | 1.190 | 1.243 | 1.469 | 1.452 |
| Example 2 | 0.791 | 0.890 | 0.916 | 0.705 |

As is clear from Table 8, in Examples 1 and 2, the distance A was less than 1.5 mm, and it was clarified that the dead space was sufficiently small. Further, it has been found that there is no possibility that the tip surface of the Luer-slip connector and the bottom surface of the recessed portion of the needle hub abut on each other even if the distance A is larger than 0.7 mm and some variation occurs, and a sufficient coupling force can be obtained by the Luer-slip connector and the needle hub.

As described above, in the recessed portion of the needle hub made of Tritan (registered trademark) having a tensile modulus of elasticity of 1550 MPa, the depth L of the recessed portion is 7.384 mm or more and 7.496 mm or less, the inner diameter D of the opening portion is 4.316 mm or more and 4.376 mm or less, and the inner diameter is formed to be narrowed from the opening portion at an inclination of 6% with respect to the depth, whereby it has been demonstrated that the distance A is reliably secured, a strong coupling force between the male Luer-slip connector and the needle hub is secured, and a dead space can be sufficiently suppressed.

As described above, in the present invention, from the viewpoint of the distance A between the tip surface of the Luer-slip connector and the bottom surface of the recessed portion of the needle hub, needle hubs made of polycarbonate, propolyprene, a cyclic olefin polymer (COP), and Tritan (registered trademark), which are thermoplastic resins having a tensile modulus of elasticity in the range of 1400 MPa or more and 2500 MPa or less, were prototyped, and diligently repeated tests and considerations. As a result, even if there are some variations in the dimensions of the earth-lip connector and the needle hub and the frictional state at the time of fitting, the distance A is reliably secured, the strong coupling force between the male Luer-slip connector and the needle hub is secured, and the dead space is suppressed.

Typically, the needle hub is dimensioned to be the value specified in Figure B.2 of ISO 80369-7: 2016 defining the dimensions of the Luer-slip connector. For example, in the case of the inner diameter D of the opening portion, the inner diameter D is determined so as to have a value of 4.198 mm or more and 4.298 mm or less. Thus, the numerical range of the present invention is outside the numerical range of the conventional needle hub to be fitted to the Luer-slip connector as defined in ISO 80369-7: 2016. A person skilled in the medical device field cannot conceive of a needle hub having dimensions outside the normal numerical range, and in fact, there has been no needle hub having dimensions within the numerical range of the present invention so far.

Examples of the tensile modulus of elasticity of polypropylene for a Luer-slip connector include 800 MPa. It is a matter well known to those skilled in the medical device field that the tensile modulus of elasticity of the Luer-slip connector is less than 1000 MPa. When the tensile modulus of elasticity A of the male Luer-slip connector is 800 MPa and the tensile modulus of elasticity B of the needle hub is 1400 MPa or more and 2500 MPa or less, the ratio B/A of the tensile modulus of elasticity B of the needle hub to the tensile modulus of elasticity A of the male Luer-slip connector is 175% or more and 312.5% or less.

When the value of the ratio B/A is less than about 175%, the hardness of the male Luer-slip connector and the hardness of the needle hub are close to each other, so that the difference in the degree of deformation between the needle hub and the male Luer-slip connector is reduced, the deformation becomes unstable, and it is difficult to obtain a certain distance A. On the other hand, when the value of the ratio B/A is more than about 312.5%, the needle hub becomes too hard as compared with the male Luer-slip connector, so that the deformation of the male Luer-slip connector becomes too large, the deformation becomes unstable, and it becomes difficult to obtain the certain distance A. By using a needle hub having a tensile modulus of elasticity in a moderate hardness range as compared with the earth-lip connector, the distance A in a certain range can be stably obtained at the time of fitting.

As described above, based on the idea of stably realizing the distance A in a certain range in consideration of the deformation of the Luer-slip connector and the needle hub at the time of fitting, the inventors prototyped needle hubs of various materials and performed a fitting test, and found the "needle hub formed of a thermoplastic resin having a tensile modulus of elasticity of 1400 MPa or more and 2500 MPa or less, and has a recessed portion having a depth L of 7.384 mm or more and 7.496 mm or less and an inner diameter D of an opening portion of 4.316 mm or more and 4.376 mm or less" of the present invention for the first time.

The description of the above-described embodiments is illustrative in all respects and is not restrictive. Modifications and changes can be made as appropriate by those skilled in the art. The scope of the present invention is defined not by the above-described embodiments but by the claims. Furthermore, the scope of the present invention includes modifications from the embodiments within the scope equivalent to the claims.

### INDUSTRIAL APPLICABILITY

The needle hub provided by the present invention makes it possible to configure various male Luer-slip connectors as defined in ISO 80369-7: 2016 and injectors.

### EXPLANATION OF REFERENCES

- 101: injector using Luer-slip connector
- 103: needle hub
- 105: syringe
- 107: plunger
- 109: front end
- 111: rear end
- 113: needle
- 115: needle receiving portion
- 117: recessed portion
- 119: opening portion
- 121: bottom surface
- 123: protruding portion
- 125: space
- 127: male thread
- 129: outer surface
- 131: inner surface
- 133: gasket
- 135: tip
- 136: injector using Luer-lock connector
- 137: syringe
- 139: outer surface
- 141: inner surface
- 143: female thread
- 145: gasket
- 147: tip

## Claims

1. A needle hub, which is formed of a thermoplastic resin having a tensile modulus of elasticity of 1400 MPa or more and 2500 MPa or less, and has a recessed portion having a conical trapezoidal shape to be fitted to a male Luer-slip connector formed of polypropylene as defined in ISO 80369-7: 2016, and in which a needle is provided on a front side of the recessed portion,
wherein the recessed portion has a depth L of 7.384 mm or more and 7.496 mm or less, an inner diameter D of an opening portion of 4.316 mm or more and 4.376 mm or less, and an inner diameter narrows from the opening portion at an inclination of 6% with respect to the depth.

2. The needle hub according to claim 1, wherein the thermoplastic resin is polycarbonate.

3. The needle hub according to claim 1, wherein the thermoplastic resin is polypropylene.

4. The needle hub according to claim 1, wherein the thermoplastic resin is a cyclic olefin polymer (COP).

5. The needle hub according to claim 1, wherein the thermoplastic resin is Tritan (registered trademark).

6. The needle hub according to any one of claims 1 to 5, wherein
the recessed portion has a smooth curved surface without unevenness, and
the curved surface generates friction between the male Luer-slip connector and the recessed portion as a whole.

7. The needle hub according to any one of claims 1 to 6, wherein
a protruding portion is formed at a substantially central position in plan view of a bottom surface of the recessed portion, and
the protruding portion is engaged with a center of the male Luer-slip connector to position a central axis and reduce a dead space between the needle hub and the male Luer-slip connector.

8. The needle hub according to any one of claims 1 to 7, wherein
a thread is provided on an outer surface of the needle hub, and
the recessed portion and the thread of the needle hub are engageable with the male Luer-lock connector as defined in ISO 80369-7: 2016.

9. An injector, comprising:
the needle hub according to any one of claims 1 to 8;
a syringe having a male Luer-slip connector as defined in ISO 80369-7: 2016; and
a plunger including a gasket at a tip,
wherein a shape of an outer surface of the gasket coincides with a shape of an inner surface of a tip of the syringe, and
a residual amount of the medical solution in the injector is 2 µL or more and 50 µL or less.
